# EUROPEAN PATENT APPLICATION

(11) **EP 2 204 128 A1**
(43) Date of publication of application: **07.07.2010**
(21) Application number: 10250005.5
(22) Date of filing: 05.01.2010
(51) Int. Cl.: A61B 17/34

(54) **Dual seal with bellows**

(30) Priority: 06.01.2009 US 142650 P; 01.12.2009 US 628609
(71) Applicant: Tyco Healthcare Group LP, North Haven, CT 06473 (US)
(72) Inventor: Abrams, Michael E., New York, NY 10282 (US); Battles, Christopher A., Seymour, CT 06483 (US); Dicesare, Paul, Easton, CT 06612 (US); Ferreira, Danial P., Milford, CT 06460 (US); Hires, Gregory R., Fairfield, CT 06824 (US)
(74) Representative: Soames, Candida Jane

(57) **Abstract**

A seal assembly which includes a housing defining a longitudinal axis, a proximal portion and a distal portion and including a bellows disposed along a length thereof. A first sealing membrane and a second sealing membrane associated with the proximal portion of the housing, each sealing membrane configured to create a substantially fluid-tight seal around a surgical Instrument inserted therethrough. The bellows configured to allow at least a portion of the housing to move radially with respect to the longitudinal axis.

## Description

### CROSS REFERENCE TO RELATED APPLICATION

The present application claims the benefit of and priority to U.S. Provisional Application Serial No. 61/142,650 filed on January 6, 2009, the entire contents of which are incorporated herein by reference.

### BACKGROUND

### Technical Field

The present disclosure relates to a seal system which is adapted to allow the introduction of surgical instrumentation into a patient's body. In particular, the present disclosure is applicable to a cannula assembly wherein a cannula housing includes or is adapted to receive a seal assembly to sealingly accommodate instruments of different diameters inserted through the seal assembly and cannula.

### Description of the Related Art

In laparoscopic procedures surgery is performed in the interior of the abdomen through a small incision; in endoscopic procedures surgery is performed in any hollow viscous of the body through narrow tubes or cannula inserted through a small entrance incision in the skin. Laparoscopic and endoscopic procedures generally require that any instrumentation inserted into the body be sealed, i.e. provisions must be made to ensure that gases do not enter or exit the body through the incision as, for example, in surgical procedures in which the surgical region is insufflated. Moreover, laparoscopic and endoscopic procedures often require the surgeon to act on organs, tissues, and vessels far removed from the incision, thereby requiring that any instruments used in such procedures be relatively long and narrow.

For such procedures, the introduction of a tube into certain anatomical cavities such as the abdominal cavity is usually accomplished by use of a trocar assembly comprised of a cannula assembly and an obturator assembly. Since the cannula assembly provides a direct passage for surgical instrumentation from outside the patient's body to access internal organs and tissue, it is important that the cannula assembly maintain a relatively gas-tight interface between the abdominal cavity and the outside atmosphere. The cannula assembly thus generally includes a cannula attached to a cannula housing containing a seal assembly adapted to maintain a seal across the opening of the cannula housing.

Since surgical procedures in the abdominal cavity of the body require insufflating gases to raise the cavity wall away from vital organs, the procedure is usually initiated by use of a Verres needle through which a gas such as CO₂ is introduced into the body cavity, thereby creating a pneumoperitoneum. Thereafter, the obturator of the obturator assembly is inserted into the cannula assembly and used to puncture the abdominal wall. The gas provides a positive pressure which raises the inner body wall away from internal organs, thereby providing the surgeon with a region within which to operate, avoiding unnecessary contact with the organs by the instruments inserted through the cannula assembly. Following removal of the obturator assembly from the cannula assembly, laparoscopic or endoscopic surgical instruments may be inserted through the cannula assembly to perform surgery within the abdominal cavity.

Without the obturator assembly to block the flow of insufflation gas out from the cavity, other structure must be provided to maintain a relatively fluid-tight interface between the abdominal cavity and the outside atmosphere. Generally in the context of insufflatory surgical procedures, there are two sealing requirements for cannula assemblies. The first requirement is to provide a substantially fluid-tight seal when an instrument is not being introduced into or is not already present in the cannula. The second requirement is to provide a substantially fluid-tight seal when an instrument is being introduced into or is already present in the cannula. Additionally, as endoscopic and laparoscopic surgical procedures and techniques have advanced, it has become desirable to accommodate surgical instrumentation of varying outside diameters through a single cannula assembly in a given surgical procedure, thereby minimizing the number of cannulae required and facilitating efficiency in the surgical procedure.

### SUMMARY

In accordance with the present disclosure a seal assembly is provided which includes a housing defining a longitudinal axis and having a proximal portion and a distal portion. The housing includes a bellows disposed along a length thereof. A first sealing membrane and a second sealing membrane associated with the proximal portion of the housing are provided. Each sealing membrane is configured to create a substantially fluid-tight seal around a surgical instrument inserted therethrough. The bellows is configured to allow at least a portion of the housing to move radially with respect to the longitudinal axis. The bellows may be configured to allow the proximal portion of the housing to move longitudinally with respect to the distal portion of the housing.

In disclosed embodiments, a distal portion of the housing is configured to mechanically engage a cannula assembly. At least one of the housing and the cannula assembly may include a sealing mechanism which is configured and dimensioned to provide a substantially fluid-tight seal in the absence of a surgical instrument passing therethrough.

The first sealing membrane may be larger than the second sealing membrane. The first sealing membrane may be adapted to receive a surgical instrument having a diameter in the range of about 8mm to about 15mm and/or the second sealing membrane may be adapted to receive a surgical instrument having a diameter in the range of about 4mm to about 8mm.

In disclosed embodiments, at least one of the first sealing membrane and the second sealing membrane is disposed in a valve. The valves may be dome valves.

A method of introducing surgical instrumentation into a patient's body is also provided, the method including the step of providing a seal assembly including a housing defining a longitudinal axis, the housing having a proximal portion and a distal portion and including a bellows disposed along a length thereof, and a first sealing membrane and a second sealing membrane associated with the proximal portion of the housing, each sealing membrane configured to create a substantially fluid-tight seal around a surgical instrument inserted therethrough. The method further includes the steps of moving the proximal portion of the housing with respect to the distal portion of the housing and inserting a first surgical instrument through the first sealing membrane such that at least a portion of the first surgical instrument is adjacent tissue.

In disclosed embodiments, the method includes the step of inserting a second surgical instrument through the second sealing membrane such that at least a portion of the second surgical instrument is adjacent tissue. The step of removing the first surgical instrument may also be included.

The step of mechanically engaging the seal assembly with a cannula assembly may also be included. The proximal portion of the housing may be moved with respect to the distal portion of the housing in a radial direction.

### BRIEF DESCRIPTION OF THE DRAWINGS

Various embodiments of the present disclosure are described herein with reference to the drawings wherein:
Fig. 1 is a longitudinal cross-sectional view of the seal assembly.
Fig. 2 is an exploded view of a cannula assembly, showing a duckbill valve fitting therein.
Fig. 3 is a longitudinal cross-sectional view of the seal assembly mounted on the cannula assembly with a surgical instrument passing therethrough.

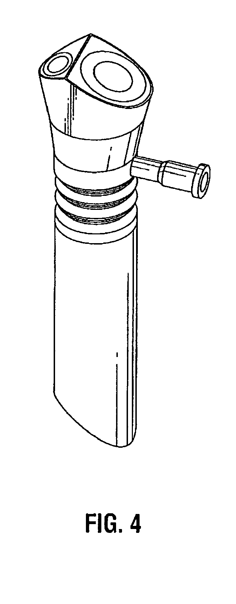

Fig 4. is a perspective view of the seal assembly.

### DETAILED DESCRIPTION

The seal assembly of the present disclosure, either alone or in combination with a cannula assembly, provides a substantial seal between a body cavity of a patient and the outside atmosphere before, during and after insertion of an instrument through the seal assembly. The seal assembly of the present disclosure is capable of accommodating instruments of varying diameters, e.g., from about 4mm to about 15mm, by providing a fluid-tight seal around each instrument when inserted through either the first valve or the second valve.

The seal assembly of the present disclosure contemplates the introduction and manipulation of various types of instrumentation adapted for insertion through a trocar and/or cannula assembly while maintaining a substantially fluid-tight interface about the instrument to preserve the atmospheric integrity of a surgical procedure from gas and/or fluid leakage. Specifically, the seal assembly incorporates a radially and longitudinally deflectable seal housing which accommodates manipulation of the surgical instrument therein. This feature of the present disclosure helps minimize the entry and exit of gases and/or fluids to/from the body cavity during manipulation of the instrumentation. Examples of instrumentation include, but are not limited to, clip appliers, graspers, dissectors, retractors, staplers, laser probes, photographic devices, endoscopes and laparoscopes, tubes, and the like. Such instruments will collectively be referred to as "instruments" or "instrumentation".

In the following description, as is traditional, the term "proximal" refers to the portion of the device closer to the operator while the term "distal" refers to the portion of the device farther from the operator.

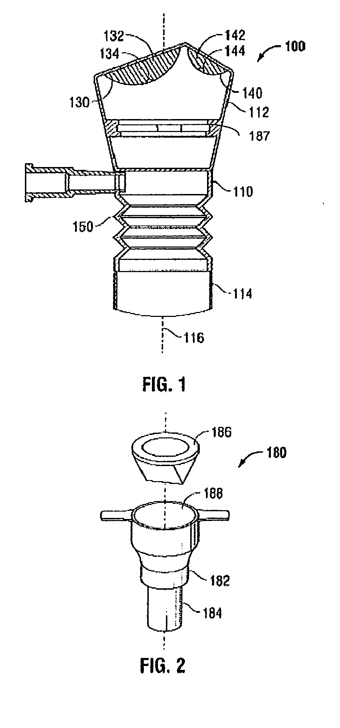

Referring now to the drawings, **Fig. 1** illustrates an access port or seal assembly **100** including housing **110** defining longitudinal axis **116.** Housing **110** has proximal portion **112** and distal portion **114.** Housing **110** includes a bellows 150 disposed along a length thereof. Bellows **150** is fabricated from a resilient material, e.g., isoprene, to facilitate the movements described hereinbelow. First valve **130** is disposed at proximal portion **112** of housing **110.** First valve **130** (e.g., a dome valve) includes sealing membrane **132.** An instrument seal **134** is defined within sealing membrane **132** to allow surgical instrumentation to pass therethrough. In disclosed embodiments, first valve **130,** sealing membrane **132** and instrument seal **134** are dimensioned and configured for receiving instruments having a diameter in the range of about 8mm to about 15mm. Sealing membrane **132** is configured to create a substantially fluid-tight seal around surgical instrumentation disposed therein. Similarly, second valve **140** includes sealing membrane **142.** An instrument seal **144** is defined within sealing membrane **142** to allow relatively small surgical instrumentation to pass therethrough. Second valve **140** may be a dome valve. In disclosed embodiments, second valve **140,** sealing membrane **142** and instrument seal **144** are dimensioned and configured for receiving instruments having a diameter in the range of about 4mm to about 8mm. Sealing membrane **142** is configured to create a substantially fluid-tight seal around surgical instrumentation disposed therein.

In a disclosed embodiment, seal assembly **100** is detachably mounted to cannula assembly **180.** As shown in **Fig. 2****,** cannula assembly **180** includes cannula housing **182** and cannula **184** which is attached thereto and which extends distally therefrom. In disclosed embodiments, cannula assembly **180** includes a sealing mechanism, such as distally directed duckbill valve **186** mounted in interior region **188** of cannula housing **182.** Duckbill valve **186** provides a substantially fluid-tight seal when communicating with an insufflated body cavity to substantially prevent escape of gases and fluids from inside the body cavity when no instrument is present in cannula assembly **180.** It is also envisioned that seal assembly **100** includes a substantially fluid-tight seal **187** (**Fig. 1**) which provides a substantially fluid-tight seal along with or in alternative to duckbill valve **186.** Dual seal assembly **100** may be associated with, or joined to, cannula assembly **180** in a variety of ways. In a disclosed embodiment, seal housing **110** of seal assembly **100** and cannula housing **182** of cannula assembly **180** are adapted to detachably engage each other, e.g., through a bayonet lock, threaded connection, or like mechanical means.

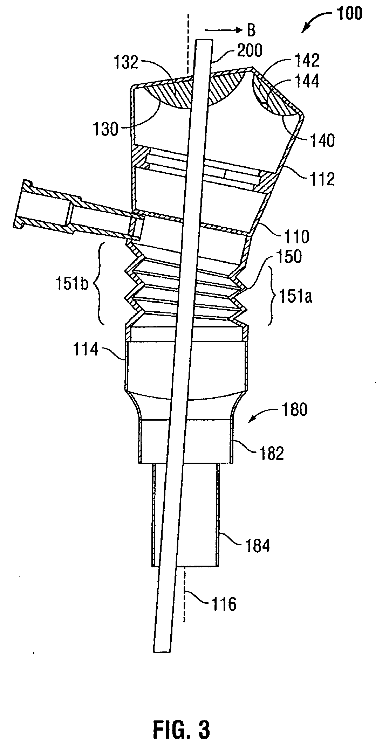

The operation of dual seal assembly **100** in conjunction with cannula assembly **180** will now be described with reference to **Figs. 1** and **3****.** Prior to insertion of a surgical instrument, duckbill valve **186** provides a fluid-tight seal within cannula assembly **180.** **Fig. 1** illustrates dual seal assembly **100** in a first or at-rest position in which bellows **150** is substantially symmetrical about longitudinal axis **116.** Seal assembly **100** may be mounted on cannula assembly **180.** In this position, seal assembly **100** can receive surgical instrumentation through first valve **130** or second valve **140,** depending on the diameter of the instrument. First valve **130** is shown as a relatively large dome valve and is configured to receive instrumentation having a diameter in the range of about 8mm to about 15mm. Second valve **140** is shown as a relatively small dome valve and is configured to receive instrumentation having a diameter in the range of about 4mm to 8mm.

An instrument is inserted into seal assembly **100** through instrument seal **134** or **144,** depending on the diameter of the instrument. Seal membranes **132** and **142,** which define instrument seals **134** and **144,** respectively, stretch to accommodate the instrument diameter passing therethrough, as necessary. The instrument **200** is advanced through instrument seal **134** or **144,** whereby a seal is created between sealing membrane **132** or **134** and the instrument passing therethrough. The instrument 200 is further advanced distally through housing **110,** cannula assembly **180,** duckbill valve **186** and into cannula **184.**

Once instrumentation is positioned as described above, via first valve **130** or second valve **140,** it may be desired to move the instrument in order to perform surgical procedures. As shown in **Fig. 3****,** when a surgeon moves instrument **200** radially in the direction of arrow **B,** away from longitudinal axis **116,** bellows **150** is compressed along the edge **151 a** facing the direction of **B** and elongated along the edge **151b** facing away from the direction of **B**. This above described action allows the proximal portion **112** of housing **110** to move radially with respect to respect to longitudinal axis **116.** Distal portion **114** of housing **110** remains relatively stationary, thereby maintaining the connection between cannula assembly **180** and housing **110.** Proximal portion **112** of housing **110** may also be moved longitudinally with respect to distal portion **114** of housing **110,** thereby maintaining the seal around surgical instrumentation disposed therein. Allowing proximal portion **112** of seal assembly **100** to move in conjunction with instrument **200** maintains the integrity of the seal around instrumentation passing therethrough. Instrumentation inserted through seal assembly **100** may ultimately be removed from seal assembly **100** by pulling the instrumentation proximally until the entire instrument is removed from seal assembly **100.**

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

## Claims

1. A seal assembly comprising:
a housing defining a longitudinal axis, the housing having a proximal portion and a distal portion and including a bellows disposed along a length thereof; and
a first sealing membrane and a second sealing membrane associated with the proximal portion of the housing, each sealing membrane configured to create a substantially fluid-tight seal around a surgical instrument inserted therethrough;
wherein the bellows is configured to allow at least a portion of the housing to move radially with respect to the longitudinal axis.

2. The seal assembly of claim 1, wherein the distal portion of the housing is configured to mechanically engage a cannula assembly.

3. The seal assembly of claim 1 or claim 2, wherein at least one of the housing and the cannula assembly includes a sealing mechanism which is configured and dimensioned to provide a substantially fluid-tight seal in the absence of a surgical instrument passing therethrough.

4. The seal assembly of any preceding claim, wherein the first sealing membrane is larger than the second sealing membrane.

5. The seal assembly of any preceding claim, wherein the first sealing membrane is adapted to receive a surgical instrument having a diameter in the range of about 8mm to about 15mm.

6. The seal assembly of any preceding claim, wherein the second sealing membrane is adapted to receive a surgical instrument having a diameter in the range of about 4mm to about 8mm.

7. The seal assembly of claim 5, wherein the second sealing membrane is adapted to receive a surgical instrument having a diameter in the range of about 4mm to about 8mm.

8. The seal assembly of any preceding claim, wherein at least one of the first sealing membrane and the second sealing membrane is disposed in a valve.

9. The seal assembly of claim 8, wherein at least one valve is a dome valve.

10. The seal assembly of any preceding claims, wherein the bellows is configured to allow the proximal portion of housing to move longitudinally with respect to the distal portion of the housing.

11. A seal assembly, the seal assembly comprising, a housing defining a longitudinal axis, the housing having a proximal portion and a distal portion and including a bellows disposed along a length thereof, and a first sealing membrane and a second sealing membrane associated with the proximal portion of the housing, each sealing membrane configured to create a substantially fluid-tight seal around a surgical instrument inserted therethrough, for use in introducing surgical instrumentation into a patients body which comprises the steps of
moving the proximal portion of the housing with respect to the distal portion of the housing; and
inserting a first surgical instrument through the first sealing membrane such that at least a portion of the first surgical instrument is adjacent tissue.

12. The use of claim 11, further comprising the step of inserting a second surgical instrument through the second sealing membrane such that at least a portion of the second surgical instrument is adjacent tissue.

13. The use of claim 11 or claim 12, further comprising the step of removing the first surgical instrument.

14. The use if claim 11, 12 or 13, further comprising the step mechanically engaging the seal assembly with a cannula assembly.

15. The use of any of claims 11 to 14, wherein the proximal portion of the housing is moved with respect to the distal portion of the housing in a radial direction.
